# EUROPEAN PATENT SPECIFICATION

(11) **EP 1 904 014 B1**
(45) Date of publication and mention of the grant of the patent: **07.09.2016**
(21) Application number: 06787048.5
(22) Date of filing: 13.07.2006
(51) Int. Cl.: A61F 15/00, A61K 33/00, A61K 33/08, A61K 33/24, A61K 33/42, A61K 33/38, A61L 26/00, A61L 15/46, A61K 45/06, C03C 3/16, C03C 4/00, A61K 33/30

(54) **WOUND DRESSING AND METHOD OF MAKING THE SAME**
WUNDVERBAND SOWIE VERFAHREN ZU DESSEN HERSTELLUNG
PANSEMENT ET PROCEDE POUR SA FABRICATION

(30) Priority: 15.07.2005 US 699500 P
(43) Date of publication of application: 02.04.2008
(73) Proprietor: Covidien LP, Mansfield, MA 02048 (US)
(72) Inventor: DAVID, Nicole, North Easton, Massachussetts, 02356 (US); FINK, David, Franklin, Massachusetts 02038 (US); TRANCHEMONTAGNE, Alain, Warwick, RI 02889 (US)
(74) Representative: Elsy, David
(86) International application number: PCT/US2006/027089
(87) International publication number: WO 2007/011612

(56) References cited:
- WO-A2-96/24364
- US-A- 5 614 006
- US-A- 6 143 318
- US-A1- 2003 180 341
- US-A1- 2004 009 144
- US-A1- 2004 082 925
- US-A1- 2004 082 925
- US-A1- 2004 142 041
- US-B1- 6 251 424
- US-B1- 6 482 444
- US-B1- 6 555 491

## Description

### BACKGROUND

A variety of wound dressings have been suggested. However, such wound dressings are not optimal for anti-microbial wound care.

### SUMMARY

Anti-microbial wound dressings, methods of making anti-microbial wound dressings and methods of treating wounds are provided.

In an exemplary embodiment, an anti-microbial wound dressing comprises a water-soluble film-forming polymer carrier; and
a water-soluble glass-encapsulated anti-microbial agent embedded in and/or coated on a surface of the film-forming polymer carrier. In an exemplary embodiment, the film-forming polymer is a water-soluble starch. In an exemplary embodiment, the water-soluble glass comprises phosphorous pentoxide and a source of anti-microbial metal cations, such as, for example, silver cations.

In an exemplary embodiment, a method of making an anti-microbial wound dressing comprises providing a water-soluble film-forming polymer carrier; and at least one of embedding within and coating on a surface of the water-soluble film-forming polymer carrier, a water-soluble glass-encapsulated anti-microbial agent. In an exemplary embodiment, the film-forming polymer is a water-soluble starch. In an exemplary embodiment, the water-soluble glass comprises phosphorous pentoxide and a source of anti-microbial metal cations, such as, for example, silver cations.

In an exemplary embodiment, a method of treating a wound comprises applying to the wound an anti-microbial wound dressing, wherein the wound dressing is comprised of a water-soluble film-forming polymer carrier and a water-soluble glass-encapsulated anti-microbial agent that is at least one of embedded within the carrier and coated on a surface of the carrier. In an exemplary embodiment, the film-forming polymer is a water-soluble starch. In an exemplary embodiment, the water-soluble glass comprises phosphorous pentoxide and a source of anti-microbial metal cations, such as, for example, silver cations.

US 6,482,444 B1 discloses silver-containing, sol-gel derived bioactive glass compositions, methods of making said compositions and uses thereof, including wound dressings and treatments with anti-microbial and/or anti-inflammatory properties.

WO 96/24364 A2 discloses an antimicrobial composition formed from a controlled release glass comprising two or more agents (metals, selenium and/or boron) for preventing or combatting wound infections.

US 2004/082925 A1 discloses a wound dressing comprising an antimicrobial agent impregnated with a fabric of several hydrophilic and hydrophobic layers.

### BRIEF DESCRIPTION OF THE DRAWINGS

FIG. 1 is a schematic of an exemplary embodiment of an anti-microbial wound dressing.
FIG. 2 is a schematic of another exemplary embodiment of an antimicrobial wound dressing.
FIG. 3 is a schematic of another exemplary embodiment of an antimicrobial wound dressing.
FIG. 4 is a schematic of another exemplary embodiment of an antimicrobial wound dressing.
FIGS. 5A and 5B are schematics of another exemplary' embodiment of an anti-microbial wound dressing.
FIGS. 6A and 6B are schematics of yet another exemplary embodiment of an anti-microbial wound dressing.

### DETAILED DESCRIPTION

Anti-microbial wound dressings are provided, which comprise a water-soluble film-forming starch polymer carrier; and a water-soluble glass-encapsulated anti-microbial agent that is at least one of embedded within the carrier and coated on a surface of the carrier, wherein the water-soluble glass-encapsulated anti-microbial agent is provided in the form of particles in a plurality of different, pre-determined size ranges in order to provide a multi-stage release profile wherein an initial release stage is provided by dissolution of particles in the smallest particle size range and at least one subsequent release stage is provided by dissolution of particles in a larger particle size range. Methods of making such wound dressings are also provided.

Examples of suitable water-soluble film-forming polymers include starches (such as "thermoplastic" starches including hydroxypropylated starches or potato, maize or rice starch treated by high pressure and humidity) combinations thereof and the like. Co-polymers of these polymers may also be used.

In exemplary embodiments, the film-forming polymer is a water-soluble or moisture-dissolving starch. One such suitable starch is sold under the trade name Velox™ by National Starch, Inc. Other suitable water-soluble or moisture-dissolving starches include starches that exhibit properties such as, for example, dissolution properties that are similar to those of a Velox™ starch.

The term "water-soluble glass" refers to any water-soluble glass or glasslike material which can be used to encapsulate an anti-microbial agent and can be embedded in and/or coated on a surface of a film-forming polymeric material suitable for use in a wound dressing. The water-soluble glass material can be in any configuration suitable for encapsulating an active agent useful for treating a wound. In exemplary embodiments, the water-soluble glass is configured to be embedded within and/or coated on a surface of a water-soluble film-forming polymeric material. Suitable configurations include, particles and the like. Examples of suitable water-soluble glass materials are described, for example, in WO-A-98/54104, WO-A-99/62834 and WO-A-99/62835 (assigned to Giltech Limited). In an exemplary embodiment, the water-soluble glass will dissolve in an aqueous environment at a rate that is slower than the water- soluble film-forming polymer.

Phosphorous pentoxide (P₂O₅) is an especially suitable glass-forming material for use in an exemplary water-soluble glass. In exemplary embodiments, the mole percentage of phosphorus pentoxide in the glass composition is less than about 85 %, preferably less than about 75 %, more preferably less than about 65 %, even more preferably less than about 60 %, and most preferably between about 30 % and about 60 %. The water-soluble glass can also include an alkali metal, an alkaline earth metal, a lanthanoid oxide, a carbonate, combinations thereof and the like, as a glass modifier. Generally, in exemplary embodiments, the mole percentage of alkali metal, alkaline earth metal, lanthanoid oxide, carbonate, combinations thereof and the like is less than about 60 %, preferably less than about 50 %, and most preferably between about 40 % and about 60 %. Boron-containing compounds such as, for example, B₂O₃ can also be used as an additive in the water-soluble glass. In exemplary embodiments, the mole percentage of boron-containing compounds is less than about 15 % preferably less than about 10 % and most preferably about 5 % or less.

Additional compounds can be added to the water-soluble glass to modify its properties, including, but not limited to, SiO₂, Al₂O₃, SO₃, a transition metal compound (e.g., a first row transition metal compound) combinations thereof and the like. Generally, the glass will release ionic species upon dissolution. The exact ionic species released depends upon die compounds added to the glass. Glasses which release aluminum ions, sulfate ions, fluorine ions combinations thereof and the like may be desirable in some circumstances.

Typically, the water-soluble glass used in exemplary embodiments comprises phosphorus pentoxide (P₂O₅) as a principal glass-former together with any one or more glass-modifying, nontoxic materials such as sodium oxide (Na₂O), potassium oxide (K₂O), magnesium oxide (MgO), zinc oxide (ZnO), calcium oxide (CaO) combinations thereof and the like. The rate at which the water-soluble glass dissolves in a fluid is determined, at least in part, by the glass composition, generally by the ratio of the glass -modifier to glass-former and by the relative proportions of the glass-modifiers in the glass. By suitable adjustment of the glass composition, the dissolution rate can be tailored to range from about 0.001 µg/cm²/hr to about 500 µg/cm²/hr, more preferably from about 0,05 µg/cm²/hr to about 250 µg/cm²/hr, more preferably from about 0.5 µg/cm²/hr to about 200 µg/cm²/hr even more preferably from about 1 µg/cm²/hr to about 100 µg/cm²/hr and most preferably from about 2 µg/cm²/hr to about 40 µg/cm²/hr. For example, certain formulations can be prepared in order to tailor the release profile so that it is suited for a particular application or need,

In exemplary embodiments, the water-soluble glass is a phosphate glass, and can comprise a source of silver ions, which may advantageously be introduced during manufacture in various forms including, for example, silver orthophosphate (Ag₃PO₄). Other metals, of course, can alternatively or additionally be present. Examples of other suitable metals that may be provided in the alternative or in addition to various forms of silver can include, but are not limited to, Cu, Mg, Zn, Ce, Mn, Bi, Se, Cs, combinations thereof and the like. Especially suitable metals include Ag, Cu, Zn and Mg. In exemplary embodiments, the glass enables
controlled release of metal and other constituents in the glass and the content of these additives can vary in accordance with conditions of use and desired rates of release. In embodiments where the water-soluble glass includes silver, the silver content can be between about 0.01 wt. % to about 90 wt. %, preferably about 0.05 wt. % to about 80 wt. %, more preferably about 0.10 wt. % to about 75 wt. %, even more preferably about 0.5 wt. % to 65 wt. %, even more preferably about 1 wt. % to about 50 wt. %, even more preferably about 2 wt. % to about 40 wt. %, even more preferably about 2 wt. % to about 30 wt. %, even more preferably about 2.5 wt. % to about 20 wt, % and most preferably about 3 wt. % to about 9 wt. %.

Although exemplary embodiments of the water-soluble glass are described herein in terms of the mole percent of oxides, of halides, and of sulfate ions, this does not imply that such chemical species are present in the glass or that they are used for the batch for the preparation of the water-soluble glass.

The mode of release is a function of time, rate and concentration. An optimum rate of release of metal ions into an aqueous environment can be tailored based upon the particular circumstances including the specific function of the released metal. Exemplary embodiments provide a means of delivering metal ions to an aqueous medium such as, for example, a wound, at a rate which will maintain a concentration of metal ions in the aqueous medium of not less than about 0.01 part per million and not greater than about 10 parts per million. Moreover, it is desirable to provide a multi-stage release including, for example, an initial, immediate release and at least one subsequent, sustained release. In exemplary embodiments, the multi-stage release can include an initial release and at least about one to about six or more subsequent release stages.

In particular cases, there may be additional requirements. For example, it may be desirable that no residue remain after the source of the metal ions is exhausted or in other cases, where the metal is made available, it will be desirable that any materials, other than the metal itself, which are simultaneously released
should be physiologically harmless. In yet other cases, it may be necessary to ensure that the pH of the resulting solution does not fall outside defined limits.

The invention provides a wound dressing that comprises both a water-soluble film-forming polymer as a carrier material and a water-soluble, anti-microbial glass composition embedded therein and/or coated on the surface of the carrier. Each material is provided in an amount effective to enable the dressing to exhibit a multi-stage release profile when applied in an aqueous environment. In an exemplary dual-stage release embodiment, the first stage can be an initial, rapid burst of anti-microbial agent and/or other active ingredient, and the second stage can be a sustained release of anti-microbial agent and/or other active ingredient, which is longer in duration but lower in concentration of anti-microbial agent and/or other active ingredient than the initial, rapid release. Applicants have found that a dual- or multi-stage release profile of this type provides a unique advantage in the treatment of wounds by providing an immediate anti-microbial effect to prevent and/or substantially inhibit the harmful effects of bacteria initially present in a wound with the initial, rapid release followed by prolonged inhibition of bacterial migration and/or growth by the sustained release over an extended period of time. Applicants believe that a dualstage or multi-stage release profile can provide optimal anti-microbial activity during the course of wound treatment

A multi-stage release profile is achieved by, inter alia, providing one or more water-soluble glass encapsulated anti-microbial agents of varying sizes. For example, an exemplary multi-stage release embodiment can be provided with water-soluble glass encapsulated anti-microbial agents in the form of particles, which are provided in a plurality of different, pre-determined particle size ranges. Each particle size range provides a different release stage. Generally, a particle having a smaller size will dissolve faster than a larger particle. Thus, particles in the smallest particle size range can provide an initial, more rapid release stage and particles in larger particle size ranges can provide subsequent, more gradual release stages. In such embodiments, the geometry and/or configuration of the water-soluble glass encapsulated anti-microbial agent can vary as long as there are different pre-determined size ranges to provide multiple,
corresponding release stages. In exemplary embodiments, the size of the particles can range from about 0.001 micron to about 100 microns, preferably from about 0.01 micron to about 75 microns, and most preferably from about 0.1 micron to about 50 microns.

In exemplary embodiments, the water-soluble film-forming polymer and water-soluble anti-microbial glass composition can be provided in the form of one or more layers to form a multi-layer laminate dressing. The number and/or order of the layers can, of course, be tailored for the intended application of the dressing, and to provide desired release profiles.

Exemplary wound dressings can, of course, include additional active ingredients or agents such as, for example, a therapeutic agent, an organoleptic agent and a pharmaceutical agent including, for example, an additional anti-microbial agent, a growth factor, an analgesic, a tissue scaffolding agent, a wound debriding agent, a hemostatic agent, an anti-thrombogenic agent, an anesthetic, an anti-inflammatory agent, an anticancer agent, a vasodilation substance, a wound healing agent, an angiogenic agent, an angiostatic agent, an immune boosting agent, a skin sealing agent, combinations thereof and the like. Although in exemplary embodiments release of these agents may occur through dissolution of the film-forming polymeric carrier and/or water-soluble glass embedded therein or coated thereupon, release of active agents may also be triggered by a variety of other means, such as, for example, electric field or signal, pH, temperature, time, pressure, moisture, bacterial cell signaling, and light including, for example, ultra-violet light, combinations thereof and the like.

Wound dressings, as described herein, can be of any type including, for example, cellulose-based dressings, foam dressings, gel dressings, non-woven dressings, composite dressings, hydrocolloid dressings, calcium alginate dressings, film dressings (e.g., a silicone and/or thermoplastic film), combinations thereof and the like.

In exemplary foam dressings, the dressing can include a liquid impervious film laminated on a side proximal from the wound. Suitable liquid-impervious materials include, but are not limited to, moisture-vapor permeable polymeric films such as, for example, synthetic organic polymers. Suitable synthetic organic polymers can include a polyurethane, a polyether-amide block copolymer, a polyether-ester block copolymer, combinations thereof and the like. The polymeric film can be made of one or more types of monomers (e.g., copolymers) or mixtures (e.g., blends) of polymers. Other breathable materials can be used such as, for example, nonwoven materials, woven materials, knit webs, porous films, foams, paper, other known backing materials, combinations thereof and the like.

In exemplary embodiments, other anti-microbial agents that can be used instead of or in addition to a source of anti-microbial metal ions include, but are not limited to, a chlorhexidine, a chlorhexadine salt, a triclosan, a polymoxin, a tetracycline, an amino glycoside (e.g., gentamicin or Tobramycin™), a rifampicin, a bacitracin, an erythromycin, a neomycin, a chloramphenicol, a miconazole, a quinolone, a penicillin, a nonoxynol 9, a fusidic acid, a cephalosporin, a mupirocin, a metronidazole, a secropin, a protegrin, a bacteriolcin, a defensin, a nitrofurazone, a mafenide, a acyclovir, a vanocmycin, a clindamycin, a lincomycin, a sulfonamide, a norfloxacin, a pefloxacin, a nalidizic acid, an oxalic acid, an enoxacin acid, a ciprofloxacin, combinations thereof and the like. In certain embodiments, a preferred anti-microbial agent can include at least one of polyhexamethylene biguanide (PHMB), a PHMB derivative such as, for example, a biodegrdable biguanide (e.g., polyethylene hexamethylene biguanide (PEHMB)), chlorhexadine gluconate, chlorohexadine hydrochloride, ethylenediaminetetraacetic acid (EDTA), variations of EDTA such as, for example, disodium EDTA or tetrasodium EDTA, combinations thereof and the like. In further exemplary embodiments, the antimicrobial agent can be PHMB.

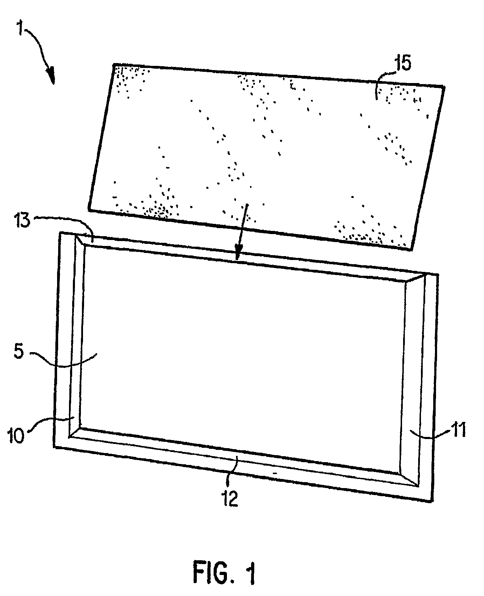

Although exemplary wound dressings can be provided in numerous configurations, exemplary configurations are depicted in FIGS. 1-6.

FIG. 1 is a schematic of an exemplary anti-microbial wound dressing 1 in the form of a non-adherent pouch 5 with a film 15 inserted therein. The pouch 5 is made of a non-adherent material such as, for example, a polyester, a polyolefin, a polyurethane, combinations thereof and the like. The pouch 5 has sealed edges 10, 11 and 12 and an open end 13. The edges 10, 11 and 12 can be sealed using any suitable means including, for example, adhesive sealing, heat sealing or thermal bonding, combinations thereof and the like. A water-soluble starch film 15 comprising a water-soluble glass-encapsulated anti-microbial agent (not shown) embedded therein and/or coated on a surface thereof can be inserted into the open end 13. Once inserted, the film 15 is positioned so that when the pouch 5 is applied to a wound the film 15 comes into contact with the wound so that dissolution of the film 15 and the water-soluble glass-encapsulated anti-microbial agent embedded therein and/or coated thereon (not shown) occurs.

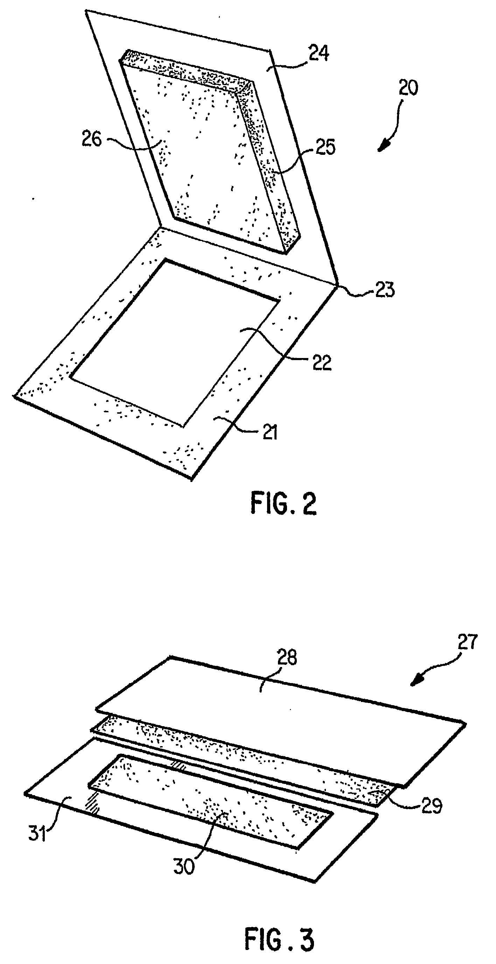

FIG. 2 is a schematic of an exemplary embodiment of a "door-style" dressing 20. The dressing 20 includes an adherent layer 21 that includes an open window 22. The adherent layer 21 can be comprised of a breathable film material such as, for example, a urethane film, a co-polyester film (e.g., a Hytrel™ film), a permeable olefin film, combinations thereof and the like. The breathable film readily provides moisture environment. The film of adherent layer 21 can be coated on one side with a skin-friendly, biocompatible adhesive such as, for example, an acrylic adhesive, a non-latex rubber adhesive, a silicone adhesive, a bio-adhesive (e.g., a denture adhesive), combinations thereof and the like. The adhesive layer 21 can also comprise a breathable composite that includes a backing film and a self-adhering biomaterial such as, for example, a hydrogel, a medical-grade foam, a hydrocolloid, combinations thereof and the like. The adhesive layer 21 can also be comprised of a non-breathable film such as a polyester film, a polyethylene film, a polypropylene film, combinations thereof and the like used together with a suitable adhesive material such as, for example, one or more of the above-described adhesive materials. Due to the possible occlusive nature of these materials, the materials may be made to include apertures or perforations to permit moisture transmission. In particular, the non-breathable film may be designed to include apertures or perforations to permit moisture transmission away from a wound.

A perforated and/or hinged portion 23 connects the adherent layer 21 to a door 24. On the door 24 is a foam pad 25. Any porous, liquid absorbent, open-cell polymeric foam material having properties that make it suitable for use as an absorbent structure can be used. Examples of suitable materials for the foam include, but are not limited to, synthetic organic polymers including, for example, polyurethane, carboxylated butadiene-styrene rubber, polyester, polyacrylate, polysaccharide, polypeptides, combinations thereof and the like. A suitable polymeric foam can be made of one or more types of monomers (e.g., copolymers) or mixtures (e.g., blends) of polymers. Often the foam used is a polyurethane foam. In addition, while suitable foams can be hydrophilic, in exemplary embodiments they can also be hydrophobic. If a hydrophobic foam material is used, the foam material can be rendered more hydrophilic with a surfactant such as, for example, a nonionic surfactant. A starch film coating 26 is applied to the foam pad 25. The starch film coating 26 includes a water-soluble glass-encapsulated anti-microbial agent coated thereon or embedded therein (not shown). The foam pad 25 and the film coating 26 are configured so that they can fit within the open window 22 in the adherent layer 21. Thus, when the door 24 is closed, the foam pad 25 and the starch film coating 26 protrude through the window 22 so that when the dressing 20 is applied to a wound, the foam pad 25 and starch film coating 26 contact the wound. In exemplary embodiments, it may be advantageous to releasably treat the non-adhesive side of the film. The releasable treatment may facilitate repeat function of the door 24 and promote clean-up of wound fluid contamination that might occur during wound care.

FIG. 3 is a schematic of an exemplary embodiment of an anti-microbial Polyskin™ or ClearWatch™, thin-film wound dressing 27. The dressing 27 includes a top delivery layer 28. The top delivery layer 28 can be made from an EVA copolymer film, a polyester, a polypropylene or a polyethylene film, combinations there of and the like, coated with a heat-activated adhesive or pressure-sensitive adhesive (PSA). Beneath the delivery layer 28 is an adhesive film dressing 29. The adhesive film dressing 29 can be comprised of a medical-grade acrylic, a silicone, a non-latex rubber, combinations thereof and the like. Beneath the adhesive film dressing 29 is a water-soluble film-forming polymeric layer 30 having a water-soluble glass-encapsulated anti-microbial agent embedded therein or coated on a surface thereof (not shown).

Covering the water-soluble film-forming polymeric film 30 is a suitable release paper 31. Before applying the dressing 27 to a wound, the release paper 31 is removed so that the water-soluble film-forming polymeric film 30 is exposed. When applied, the dressing 27 can be adhered to the skin surrounding the wound using the adhesive film dressing 29. Once applied, the anti-microbial film 30 is in contact with the wound thereby promoting dissolution of the water-soluble film-forming polymeric film 30 and the water-soluble glass-encapsulated anti-microbial agent embedded therein and/or coated on a surface thereof.

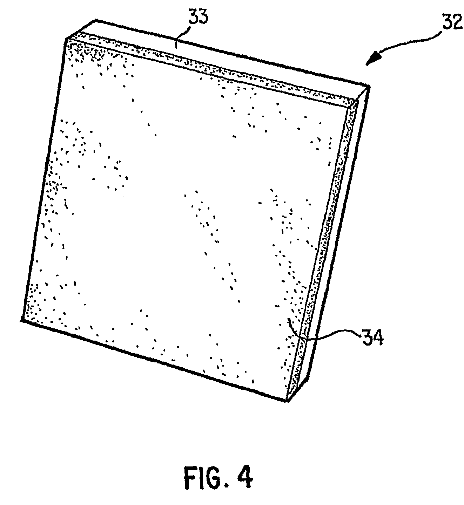

FIG. 4 is a schematic of an exemplary embodiment of an anti-microbial COPA™ with film wound dressing 32. The dressing 32 includes a foam base 33. The foam base can be of any suitable foam material including, for example, the materials described above in paragraph [0032]. On the wound side of the foam base 33 is a water-soluble film-forming polymeric film coating 34 which can include a water-soluble glass-encapsulated anti-microbial agent embedded therein and/or coated on a surface thereof (not shown). When applied to a wound, the film coating 34 and the water-soluble glass anti-microbial agent provided therein and/or coated thereupon dissolve.

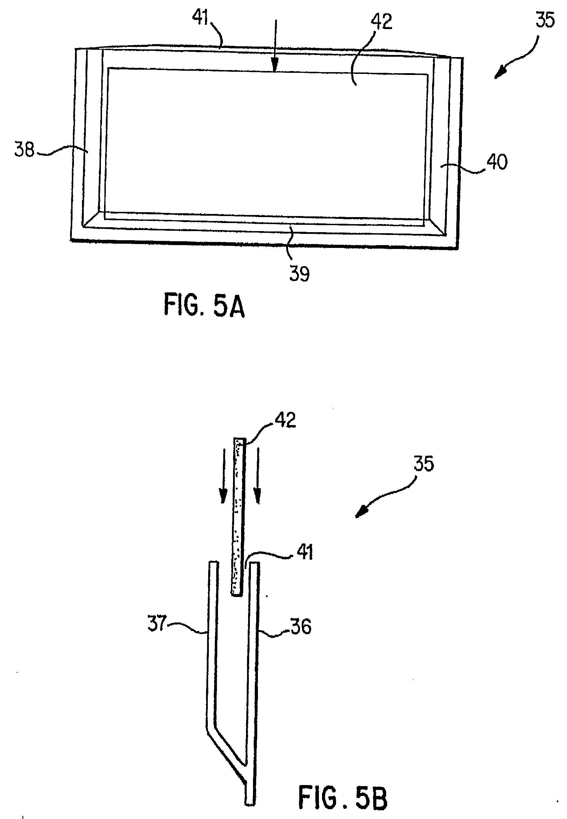

FIGS. 5A and 5B are schematics of an exemplary embodiment of an anti-microbial Telfa® pocket with film wound dressing 35. The dressing 35 includes a backing 36 made of any suitable wound dressing material such as, for example, a polymeric film material. One example of a suitable polymeric film material is polyester Mylar® from DuPont®. Other suitable polymeric film materials include, but are not limited to, a silicone rubber, a polyolefin, a polyvinylchloride, an acetate, a urethane, combinations thereof and the like. On the wound side of the dressing 35 is a non-adherent layer 37 made of a suitable wound dressing material such as, for example, a polymeric film material. Suitable polymeric film materials include, for example, polyester, polyethylene, polypropylene, combinations thereof and the like. The non-adherent layer 37 is connected to the backing 36 to form three sealed edges 38, 39 and 40. Edges 38, 39 and 40 can be sealed using any suitable means including heat sealing or thermal bonding, adhesive sealing, combinations thereof and the like. The remaining side of the dressing 35 is an open end 41. A water-soluble film-forming polymeric film 42 having a water-soluble glass-encapsulated anti-microbial agent embedded therein and/or coated on a surface thereof (not shown) is then inserted into the open end 41 so that the film 42 is centrally positioned between the backing 36 and non-adherent layer 37.

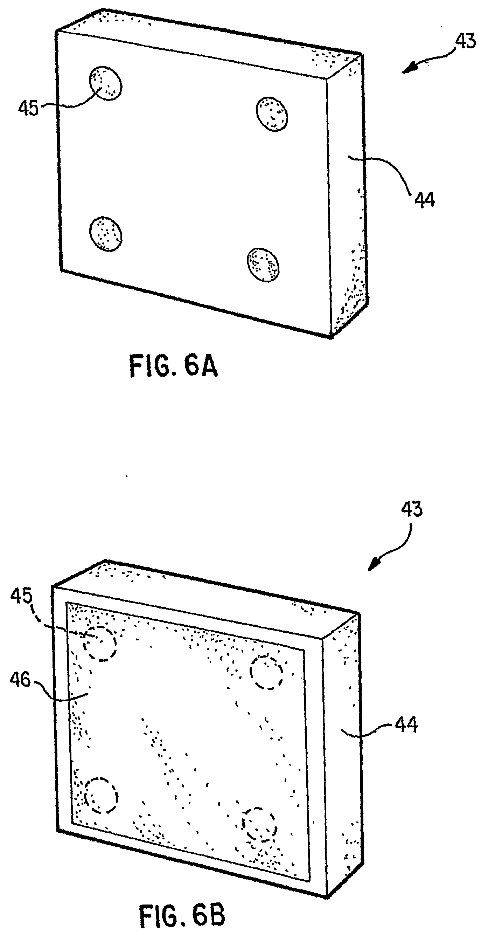

FIGS. 6A and 6B are schematics of an exemplary embodiment of an anti-microbial COPA™ dressing 43. The dressing 43 includes a foam layer 44. Any suitable foam material for use in a wound dressing can be used including, for example, the foam materials described above in paragraph [0032]. On the wound side of the foam layer 44 are a plurality of adhesive tabs 45. Although there are four adhesive tabs 45 depicted in FIGS. 6A and 6B, any number of adhesive tabs 45 can be used. In addition, in certain embodiments it may be possible to include a single adhesive tab 45. Of course, any suitable adhesive material acceptable for use in wound care may be used for the adhesive tabs 45 including, for example, the adhesive materials described in paragraphs [0031] and/or [0033]. Adhered to the adhesive tabs 45 is a water-soluble film-forming polymeric film 46, which includes a water-soluble glass-encapsulated anti-microbial agent embedded therein and/or coated on a surface thereof (not shown). In use, the dressing 43 is applied to a wound so that the water-soluble film-forming polymeric film 46 is in contact with the wound so that the film 46 and the water-soluble glass-encapsulated anti-microbial agent embedded therein or coated thereon dissolve.

### EXAMPLES

### Example 1: Water-soluble glass-encapsulated anti-microbial agents:

An anti-microbial water-soluble glass composition for use in an anti-microbial wound dressing is prepared. The composition is an inorganic polymer composed of at least one of a sodium, a calcium, a phosphorus, a zinc and a silver oxide. The water-soluble glass composition is formulated in the form of a matrix best described as an inorganic water-soluble polymer wherein the dissolution rate is determined, at least in part, by its chemical composition. The glass matrix, which is available under the trade name Corgleaes™, can be modified to provide controlled release with different delivery profiles. Based on the ratio of chemical constituents that comprise the matrix, the rate of dissolution is controlled. Particle size, which is more of a processing control, can also alter the rate of dissolution. Upon exposure to an aqueous environment, the network bonding oxygen bridges are hydrolyzed, dissolving the matrix and releasing constituents in ionic form into the surrounding environment.

The water-soluble glass composition includes one or more anti-microbial agents including silver and/or possibly at least one of copper, zinc or other antiseptic agents,

The anti-microbial agent, which can be comprised of silver in the form of silver oxide, forms an integral part of the inorganic compound's molecular structure. The polymer former establishes a chemically-ordered network of phosphorus with bridging oxygen atoms. The ratio of network modifiers (sodium, calcium and zinc) determines the rate at which the polymer dissolves by depolymerizing (i.e., hydrolyzing) the oxygen-bridging network. It is not known if there is a preferential hydrolysis of specific bonds, however, the polymer dissolves horn the outside inward and does not leach ions until they become exposed. Testing indicates that the calcium and zinc proportions in the polymer strengthen or shield the bridging oxygen from hydrolysis thereby reducing the rate of dissolution. Increasing the proportion of Group I oxides (such as sodium) facilitates hydrolysis thus increasing the rate of dissolution of the polymer.

The water-soluble glass-encapsulated anti-microbial agent has a dissolution rate in the range of about 0.001 µg/cm²/hr to about 500 µg/cm²/hr, preferably to about 2 µg/cm²/hr to about 40 µg/cm²/hr. Additionally, the water-soluble glass- encapsulated anti-microbial agent includes about 0.1 wt. % to about 90 wt. % silver, preferably about 3 wt. % to about 9 wt. % silver

Exemplary water-soluble glass-encapsulated anti-microbial agent compositions are described in Table I presented below.

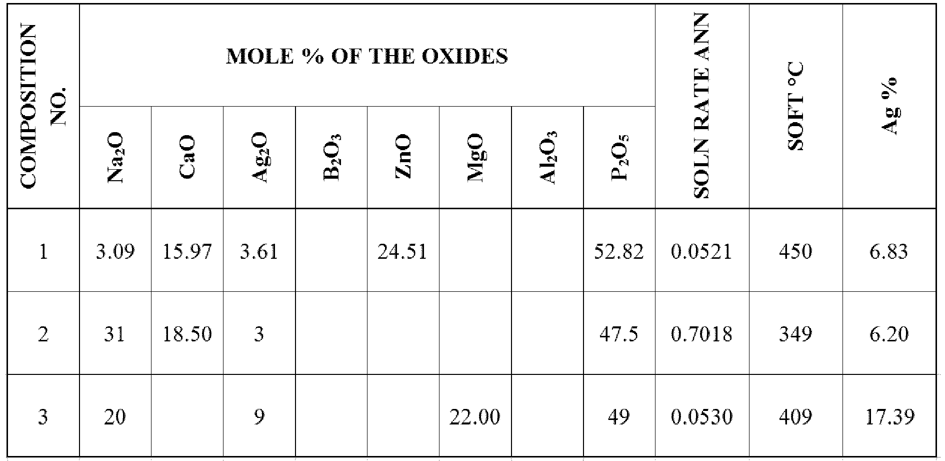

### EXAMPLE 2: Water-soluble film-forming polymeric carrier:

A water-soluble film-forming polymeric carrier is prepared using a hydrophilic starch polymer. One starch is provided by National Starch and Chemical Corporation under the trade name Velox™. The starch dissolves in aqueous environments because it is modified to ensure rapid solubility upon contact with an aqueous environment. Applicants find that Velox™ starch films can have a faster dissolution rate than other film-forming polymers. Of course, other commercially-available water-soluble or moisture-dissolvable starches having similar properties including, for example, dissolution properties, can be used instead of or in addition to a Velox™ starch. For example, one or more of the following moisture-dissolvable starches or polymers can be used instead of or in addition to Velox™ starch: corn starch, can be used as a surface coating or as an adhesive in the dextrin form, which is a roasted starch; hydroxypropyl methylcellulose (HPMC), which is cellulose ether that is derived from alkali treated cellulose (it can be used as an alternative to gelatin for encapsulating actives); pectin, which is a heterosaccharide derived from a plant cell wall (in certain conditions the pectin can form a gel and is also used as an oral demulcent which will dissolve in wet conditions); and pullulan, which is an extracellular bacterial polysaccharide produced from starch (pullulan is very soluble in water and also has film forming abilities). Other examples include, but are not limited to, Tapioca starch, sodium alginates and cyclodextrin.

To evaluate the dissolution rate of a suitable moisture-dissolvable film, a study is conducted wherein the dissolution rate of various film-forming polymers is determined by placing the film-forming polymers (e.g., 10 x 10 mm) in water at about ambient temperature or about 37°C. The film-forming polymers tested are presented in Table II.

**Table II**

| Film-Forming Polymers |
|---|
| Polymer |
| Velox™ Starch |
| Corn Starch |
| Tapioca Starch |
| Hydroxypropyl methyl cellulose (Adhesives Research) |
| Pectin |
| Pullulan (LTS Lohmann) |
| Sodium Alginate (Watson Foods) |
| Cavamax® Cyclodextrins (ISP) |

| |
|---|
| * Hydroxypropyl methylcellulose (Dissolution test conducted in about 37°C water). |

The dissolution test is conducted by taking a piece of film/dressing and immersing it in DI water at about 37 °C. The film/dressing is periodically removed and placed in fresh DI water. Water samples are collected and analyzed for silver content using inductively coupled plasma (ICP), inductively coupled plasma mass spectroscopy (ICP-MS), or atomic absorption (AA). DI water can be replaced by other media such as, for example, simulated wound fluid, serum, combinations thereof and the like.

An alternative test can be conducted by taking a piece of dressing and immersing it in DI water at a temperature of about 37 °C. The volume of DI water used in this test is generally greater than the volume of DI water used in the above-described test. Periodically, a volume of DI water is analyzed for silver content using ICP, ICP-MS or AA. The removed volume of DI water is replaced with fresh DI water and a dilution factor is applied in the calculations. Again, the DI water can be replaced with other media such as, for example, simulated wound fluid, serum, combinations thereof and the like.

Yet another test includes taking a piece of dressing and placing it on an inoculated agar plate. An agar plate is a microbiological culture medium that contains at least one source of nutrients such as, for example, gram positive bacteria, gram negative bacteria, yeast or fungi. Examples of suitable sources include, but are not limited to, S. aureus, P. aeruginisa, Candida albicans, combinations thereof and the like. The consistency of a liquid medium is modified with the use of agar to change the medium into a solid or semisolid state. The agar concentration is varied from about 0.5% to about 5%, preferably about 1% to about 4.5%, and most preferably about 2% to about 4%. An agar medium of less than about 1% is mostly liquid. In addition to agar, the culture medium can include blood, serum, albumin, combinations thereof and the like, depending on the nutrient requirements. The plate is incubated at about 37 °C. At a selected time, the dressing is removed from the agar plate and placed on a fresh agar plate. The agar below the dressing is biopsyed and analyzed for silver content. The dressing is continuously moved to fresh agar plates for a selected period of time.

Alternatively, the total silver content of a piece of dressing can be determined using an extraction method. Additional steps such as those described in the test of paragraph [0048] can also be performed. Instead of an agar biopsy, the change in silver content in the dressing is determined using the same extraction method used to determine the total initial silver content.

The dissolution test can also be done using a method wherein the original content of silver contained in the film/dressing is determined and the amount of silver reduction over time (e.g., about 24 hours) is measured. After this determination, the steps such as those described above can be performed.

An elution analysis can also be conducted to determine the rate of silver release. The film/dressing is placed in an agar solution (about 0.5% to about 1% agar) or in a simulated wound serum. The solution is tested by ICP-MS. The exact method of analysis is determined depending on the test solution used.

A test can also be conducted to determine the activity against organisms. In one test, a piece of dressing is immersed in a phosphate buffer at a temperature of about 37 °C. A test organism (inoculum) is added to the solution. After a selected period of time, changes in the test organism counts are assessed by plating and incubation for about 24 hours. The plate count procedure includes an anti-microbial neutralization step. The results obtained are compared with control samples that can include no dressing or a plain dressing.

Another such test is conducted by evaluating changes in the zone of inhibition (ZOI). A piece of dressing is placed on an agar plate (about 1% to about 4% agar). The plate is inoculated with a test organism. The development of the ZOI surrounding the dressing is observed. The development of the ZOI indicates that silver has eluted into the agar resulting in inhibition of the test organism growth. Alternately, at selected points in time, the dressing is placed on a fresh agar plate and changes in the ZOI development are assessed.

Yet another efficacy test can include taking a piece of dressing and placing it on an agar plate inoculated with a test organism. After a certain period of time, the dressing is removed and the agar plug below the dressing is biopsied. The agar plug is broken up into phosphate buffer and a neutralizing solution. Organism counts are obtained by plating. An agar plate is exposed to a non-anti-microbial dressing or no dressing to form a control. The results of the test and control samples are compared to assess the efficacy of the anti-microbial dressing.

During the study, it is found that thicker Velox™ or substantially equivalent starch films still exhibit faster dissolution properties than other film-forming polymers at similar conditions. The Velox™ film's faster dissolution rate is beneficial in exemplary embodiments where it is desirable to provide an initial rapid burst of anti-microbial agent, which can be obtained by the rapid dissolution of the Velox™ starch film followed by a second, sustained anti-microbial release resulting from the dissolution of a water-soluble glass-encapsulated anti-microbial agent, which is either embedded within the Velox™ film and/or coated on a surface thereof. Again, exemplary embodiments can achieve these advantages using other suitable moisture-dissolvable starch films including, but not limited to, one or more of the following starches instead of or in addition to Velox™: corn starch, tapioca starch, HPMC, pectin, pullulan, sodium alginate, cyclodextrin, combinations thereof and the like. Although a faster dissolution rate may be desirable in certain circumstances, it is, of course, not required.

Applicants modify the Velox™ or equivalent starch film chemically and/or physically. The Velox™ or equivalent film can be composed of multiple starches in order to achieve a desired balance of properties.

## Claims

1. An anti-microbial wound dressing comprising a water-soluble film-forming starch polymer carrier; and a water-soluble glass-encapsulated anti-microbial agent that is at least one of embedded within the carrier and coated on a surface of the carrier, wherein the water-soluble glass-encapsulated anti-microbial agent is provided in the form of particles in a plurality of different, pre-determined size ranges in order to provide a multi-stage release profile wherein an initial release stage is provided by dissolution of particles in the smallest particle size range and at least one subsequent release stage is provided by dissolution of particles in a larger particle size range.

2. The wound dressing of Claim 1, wherein the anti-microbial agent encapsulated in the water-soluble glass is a metal cation.

3. The wound dressing of Claim 1, wherein the anti-microbial agent comprises at least one of a sodium oxide, a calcium oxide, a phosphorus oxide, a zinc oxide, a silver oxide, copper oxide, boron oxide, and combinations thereof.

4. The wound dressing of Claim 1, further comprising at least one additional active agent selected from the group consisting of a growth factor, an analgesic, a hemostatic agent, an anti-thrombogenic agent, an anesthetic, an anti-inflammatory agent, an anticancer agent, a vasodilation substance, a wound healing agent, an angiogenic agent, an angiostatic agent, an immune boosting agent, a buffering compound, a tissue scaffolding agent, another anti-microbial agent, a skin sealing agent, a wound debriding agent, an anti-microbial agent selected from the group consisting of a chlorhexidine, a chlorhexadine salt, a triclosan, a polymoxin, a tetracycline, an amino glycoside, a rifampicin, a bacitracin, an erythromycin, a neomycin, a chloramphenicol, a miconazole, a quinolone, a penicillin, a nonoxynol 9, a fusidic acid, a cephalosporin, a mupirocin, a metronidazole, a secropin, a protegrin, a bacteriolcin, a defensin, a nitrofurazone, a mafenide, a acyclovir, a vanocmycin, a clindamycin, a lincomycin, a sulfonamide, a norfloxacin, a pefloxacin, a nalidizic acid, an oxalic acid, an enoxacin acid, a ciprofloxacin, combinations thereof and the like.

5. The wound dressing of Claim 1, wherein the anti-microbial agent is selected from the group consisting of PHMB, a PHMB derivative which is a biodegradable biguanide, and PEHMB.

6. A method of making an anti-microbial wound dressing, the method comprising providing a water-soluble film-forming starch polymer carrier; at least one of embedding within or coating on a surface of the water-soluble film-forming polymer carrier, a water-soluble glass-encapsulated anti-microbial agent, wherein the water-soluble glass-encapsulated anti-microbial agent is provided in the form of particles in a plurality of different, pre-determined size ranges in order to provide a multi-stage release profile wherein an initial release stage is provided by dissolution of particles in the smallest particle size range and at least one subsequent release stage is provided by dissolution of particles in a larger particle size range.

7. The method of Claim 6, wherein the amount of water-soluble film-forming polymer carrier and the amount of water-soluble glass-encapsulated anti-microbial agent embedded therein and/or coated thereon are effective to provide a first release of anti-microbial agent and at least one subsequent, sustained release of anti-microbial agent, wherein the first release is shorter in duration but higher concentration of anti-microbial agent than the at least one subsequent, substained release.

8. The method of Claim 7, wherein the anti-microbial agent encapsulated in the water-soluble glass is a metal cation.

9. The method of Claim 7, wherein the anti-microbial agent comprises at least one of a sodium oxide, a calcium oxide, a phosphorus oxide, a zinc oxide, a silver oxide, copper oxide, boron oxide, and combinations thereof.

10. The method of Claim 7, further comprising incorporating at least one additional active agent into the carrier and/or water-soluble glass, wherein the at least one additional active agent is selected from the group consisting of a growth factor, an analgesic, a hemostatic agent, an anti-thrombogenic agent, an anesthetic, an anti-inflammatory agent, an anticancer agent, a vasodilation substance, a wound healing agent, an angiogenic agent, an angiostatic agent, an immune boosting agent, a buffering compound, a tissue scaffolding agent, another anti-microbial agent, a wound debriding agent, and an anti-microbial agent selected from the group consisting of a chlorhexidine, a chlorhexadine salt, a triclosan, a polymoxin, a tetracycline, an amino glycoside, a rifampicin, a bacitracin, an erythromycin, a neomycin, a chloramphenicol, a miconazole, a quinolone, a penicillin, a nonoxynol 9, a fusidic acid, a cephalosporin, a mupirocin, a metronidazole, a secropin, a protegrin, a bacteriolcin, a defensin, a nitrofurazone, a mafenide, a acyclovir, a vanocmycin, a clindamycin, a lincomycin, a sulfonamide, a norfloxacin, a pefloxacin, a nalidizic acid, an oxalic acid, an enoxacin acid, a ciprofloxacin, combinations thereof and the like.

11. The method of Claim 7, wherein the anti-microbial agent is selected from the group consisting of PHMB, a PHMB derivative which is a biodegradable biguanide, and PEHMB.

## Patentansprüche

1. Antimikrobieller Wundverband, umfassend einen wasserlöslichen filmbildenden Stärkepolymerträger; und einen wasserlöslichen glasverkapselten antimikrobiellen Wirkstoff, der wenigstens eins von eingebettet in den Träger und auf eine Oberfläche des Trägers aufgebracht ist, wobei der wasserlösliche glasverkapselte antimikrobielle Wirkstoff in der Form von Partikeln in einer Vielzahl von verschiedenen vorgegebenen Größenbereichen bereitgestellt wird, um ein mehrstufiges Freisetzungsprofil bereitzustellen, wobei eine erste Freisetzungsstufe durch Lösen der Partikel im kleinsten Partikelbereich bereitgestellt wird und wenigstens eine nachfolgende Freisetzungsstufe durch Lösen von Partikeln in einem größeren Partikelgrößenbereich bereitgestellt wird.

2. Wundverband nach Anspruch 1, wobei der antimikrobielle Wirkstoff, der in dem wasserlöslichen Glas verkapselt ist, ein Metallkation ist.

3. Wundverband nach Anspruch 1, wobei der antimikrobielle Wirkstoff wenigstens eins von einem Natriumoxid, einem Calciumoxid, einem Phosphoroxid, einem Zinkoxid, einem Silberoxid, Kupferoxid, Boroxid und Kombinationen davon umfasst.

4. Wundverband nach Anspruch 1, ferner umfassend wenigstens einen weiteren aktiven Wirkstoff, ausgewählt aus der Gruppe bestehend aus einem Wachstumsfaktor, einem Analgetikum, einem hämostatischen Wirkstoff, einem Antithrombosewirkstoff, einem Anästhetikum, einem entzündungshemmenden Wirkstoff, einem Antikrebswirkstoff, einem blutgefäßerweiternden Stoff, einem Wundheilungswirkstoff, einem angiogenen Wirkstoff, einem angiostatischen Wirkstoff, einem die Immunabwehr verstärkenden Wirkstoff, einer Pufferverbindung, einem Gewebe-Scaffolding-Wirkstoff, einem weiteren antimikrobiellen Wirkstoff, einem hautverschließenden Wirkstoff, einem Wunddebridement-Wirkstoff, einem antimikrobiellen Wirkstoff ausgewählt aus der Gruppe bestehend aus einem Chlorhexidin, einem Chlorhexadinsalz, einem Triclosan, einem Polymoxin, einem Tetracyclin, einem Aminoglycosid, einem Rifampicin, einem Bacitracin, einem Erythromycin, einem Neomycin, einem Chloramphenicol, einem Miconazol, einem Chinolon, einem Penicillin, einem Nonoxynol 9, einer Fusidinsäure, einem Cephalosporin, einem Mupirocin, einem Metronidazol, einem Secropin, einem Protegrin, einem Bacteriolcin, einem Defensin, einem Nitrofurazon, einem Mafenid, einem Acyclovir, einem Vancomycin, einem Clindamycin, einem Lincomycin, einem Sulfonamid, einem Norfloxacin, einem Pefloxacin, einer Nalidixinsäure, einer Oxalsäure, einer Enoxacinsäure, einem Ciprofloxacin, Kombinationen davon und dergleichen.

5. Wundverband nach Anspruch 1, wobei der antimikrobielle Wirkstoff ausgewählt ist aus der Gruppe bestehend aus PHMB, einem PHMB-Derivat, das ein biologisch abbaubares Biguanid ist, und PEHMB.

6. Verfahren zum Herstellen eines antimikrobiellen Wundverbands, wobei das Verfahren Folgendes umfasst: Bereitstellen eines wasserlöslichen filmbildenden Stärkepolymerträgers; wenigstens eins von Einbetten eines wasserlöslichen glasverkapselten antimikrobiellen Wirkstoffs in eine Oberfläche des wasserlöslichen filmbildenden Polymerträgers oder Aufbringen des wasserlöslichen glasverkapselten antimikrobiellen Wirkstoffs auf die Oberfläche des wasserlöslichen filmbildenden Polymerträgers, wobei der wasserlöslichen glasverkapselte antimikrobielle Wirkstoff in der Form von Partikeln in einer Vielzahl von verschiedenen vorgegebenen Größenbereichen bereitgestellt wird, um ein mehrstufiges Freisetzungsprofil bereitzustellen, wobei eine erste Freisetzungsstufe durch Lösen der Partikel im kleinsten Partikelbereich bereitgestellt wird und wenigstens eine nachfolgende Freisetzungsstufe durch Lösen von Partikeln in einem größeren Partikelgrößenbereich bereitgestellt wird.

7. Verfahren nach Anspruch 6, wobei die Menge des wasserlöslichen filmbildenden Polymerträgers und die Menge des darin eingebetteten und/oder darauf aufgebrachten wasserlöslichen glasverkapselten antimikrobiellen Wirkstoff wirksam ist, um eine erste Freisetzung von antimikrobiellem Wirkstoff und wenigstens eine nachfolgende kontinuierliche Freisetzung von antimikrobiellem Wirkstoff bereitzustellen, wobei die erste Freisetzung eine kürzere Dauer, aber eine höhere Konzentration des antimikrobiellen Wirkstoffs aufweist als die wenigstens eine nachfolgende kontinuierliche Freisetzung.

8. Verfahren nach Anspruch 7, wobei der antimikrobielle Wirkstoff, der in dem wasserlöslichen Glas verkapselt ist, ein Metallkation ist.

9. Verfahren nach Anspruch 7, wobei der antimikrobielle Wirkstoff wenigstens eins von einem Natriumoxid, einem Calciumoxid, einem Phosphoroxid, einem Zinkoxid, einem Silberoxid, Kupferoxid, Boroxid und Kombinationen davon umfasst.

10. Verfahren nach Anspruch 7, ferner umfassend Integrieren von wenigstens einem weiteren aktiven Wirkstoff in den Träger und/oder das wasserlösliche Glas, wobei der wenigstens eine weitere aktiven Wirkstoff ausgewählt wird aus der Gruppe bestehend aus einem Wachstumsfaktor, einem Analgetikum, einem hämostatischen Wirkstoff, einem Antithrombosewirkstoff, einen Anästhetikum, einem entzündungshemmenden Wirkstoff, einem Antikrebswirkstoff, einem blutgefäßerweiternden Stoff, einem Wundheilungswirkstoff, einem angiogenen Wirkstoff, einem angiostatischen Wirkstoff, einem die Immunabwehr verstärkenden Wirkstoff, einer Pufferverbindung, einem Gewebe-Scaffolding-Wirkstoff, einem weiteren antimikrobiellen Wirkstoff, einem Wunddebridement-Wirkstoff und einem antimikrobiellen Wirkstoff ausgewählt aus der Gruppe bestehend aus einem Chlorhexidin, einem Chlorhexadinsalz, einem Triclosan, einem Polymoxin, einem Tetracyclin, einem Aminoglycosid, einem Rifampicin, einem Bacitracin, einem Erythromycin, einem Neomycin, einem Chloramphenicol, einem Miconazol, einem Chinolon, einem Penicillin, einem Nonoxynol 9, einer Fusidinsäure, einem Cephalosporin, einem Mupirocin, einem Metronidazol, einem Secropin, einem Protegrin, einem Bacteriolcin, einem Defensin, einem Nitrofurazon, einem Mafenid, einem Acyclovir, einem Vancomycin, einem Clindamycin, einem Lincomycin, einem Sulfonamid, einem Norfloxacin, einem Pefloxacin, einer Nalidixinsäure, einer Oxalsäure, einer Enoxacinsäure, einem Ciprofloxacin, Kombinationen davon und dergleichen.

11. Verfahren nach Anspruch 7, wobei der antimikrobielle Wirkstoff ausgewählt wird aus der Gruppe bestehend aus PHMB, einem PHMB-Derivat, das ein biologisch abbaubares Biguanid ist, und PEHMB.

## Revendications

1. Pansement antimicrobien comprenant un film hydrosoluble formant un support pour polymère d'amidon ; et un agent antimicrobien encapsulé dans du verre hydrosoluble qui est au moins un de ceux logés dans le support et enduit sur une surface du support, l'agent antimicrobien encapsulé dans du verre hydrosoluble étant utilisé sous la forme de particules dans une pluralité de gammes de tailles différentes, prédéfinies, afin de procurer un profil de libération multiétage dans lequel un stade initial de libération a lieu par dissolution de particules dans la plus petite gamme de taille de particule et au moins un stade consécutif de libération a lieu par dissolution de particules dans une plus grande gamme de taille de particule.

2. Pansement selon la revendication 1, dans lequel l'agent antimicrobien encapsulé dans le verre hydrosoluble est un cation métallique.

3. Pansement selon la revendication 1, dans lequel l'agent antimicrobien comprend au moins soit un oxyde de sodium, un oxyde de calcium, un oxyde de phosphore, un oxyde de zinc, un oxyde d'argent, un oxyde de cuivre, un oxyde de bore et leurs combinaisons.

4. Pansement selon la revendication 1, comprenant en outre au moins un agent actif supplémentaire choisi dans l'ensemble constitué d'un facteur de croissance, d'un analgésique, d'un agent hémostatique, d'un agent anti-thrombogénique, d'un anesthésique, d'un agent anti-inflammatoire, d'un agent anticancer, d'une substance de vasodilatation, d'un agent de cicatrisation de plaie, d'un agent angiogénique, d'un agent angiostatique, d'un agent de stimulation de l'immunité, d'un composé de tamponnage, d'un agent d'échafaudage de tissu, d'un autre agent antimicrobien, d'un agent de scellement de peau, d'un agent de débridement de plaie, d'un agent antimicrobien choisi dans l'ensemble constitué de chlorhexidine, d'un sel de chlorhexidine, d'un triclosane, d'une polymoxine, d'une tétracycline, d'un amino glycoside, d'une rifampicine, d'une bacitracine, d'une érythromycine, d'une néomycine, d'un chloramphénicol, d'une miconazole, d'une quinolone, d'une pénicilline, d'un nonoxynol 9, d'un acide fusidique, d'une céphalosporine, d'une mupirocine, d'une métronidazole, d'une sécropine, d'une protégrine, d'une bactériolcine, d'une défensine, d'une nitrofurazone, d'un mafénide, d'un acyclovir, d'une vancomycine, d'une clindamycine, d'une lincomycine, d'un sulfonamide, d'une norfloxacine, d'une pefloxacine, d'un acide nalidizique, d'un acide oxalique, d'un acide d'énoxacine, d'une cipro-floxacine, de leurs combinaisons et similaires.

5. Pansement selon la revendication 1, dans lequel l'agent antimicrobien est choisi dans l'ensemble constitué de PHMB, d'un dérivé de PHMB qui est un biguanide biodégradable et de PEHMB.

6. Procédé de fabrication d'un pansement antimicrobien, le procédé comprenant l'utilisation d'un support de polymère d'amidon formant un film et hydrosoluble ; au moins un logement dans une surface du support de polymère formant un film et hydrosoluble ou l'enduisant, un agent antimicrobien encapsulé dans du verre hydrosoluble, l'agent antimicrobien encapsulé dans du verre hydrosoluble étant utilisé sous la forme de particules dans une pluralité de gammes de taille différentes, prédéfinies, afin d'obtenir un profil multiétage de libération où un étage initial de libération a lieu par dissolution de particules dans la gamme de taille de particule la plus petite et au moins un étage suivant de libération a lieu par dissolution de particules dans une gamme de taille plus grande de particule.

7. Procédé selon la revendication 6, dans lequel la quantité de support de polymère formant un film et hydrosoluble et la quantité d'agent antimicrobien encapsulé dans du verre hydrosoluble qui y sont logés et/ou enduits dessus sont efficaces pour donner lieu à une première libération d'agent antimicrobien et au moins une libération suivante et soutenue d'agent antimicrobien, la première libération durant moins longtemps mais en plus forte concentration de l'agent antimicrobien que l'au moins une libération suivante et soutenue.

8. Procédé selon la revendication 7 dans lequel l'agent antimicrobien encapsulé dans le verre hydrosoluble est un cation métallique.

9. Procédé selon la revendication 7 dans lequel l'agent antimicrobien comprend au moins soit un oxyde de sodium, un oxyde de calcium, un oxyde de phosphore, un oxyde de zinc, un oxyde d'argent, un oxyde de cuivre, un oxyde de bore et leurs combinaisons.

10. Procédé selon la revendication 7, comprenant en outre l'incorporation d'au moins un agent actif supplémentaire dans le support et/ou le verre hydrosoluble, l'au moins un agent actif supplémentaire étant choisi dans l'ensemble constitué d'un facteur de croissance, d'un analgésique, d'un agent hémostatique, d'un agent anti-thrombogénique, d'un anesthésique, d'un agent anti-inflammatoire, d'un agent anticancer, d'une substance de vasodilatation, d'un agent de cicatrisation de plaie, d'un agent angiogénique, d'un agent angiostatique, d'un agent de stimulation de l'immunité, d'un composé de tamponnage, d'un agent d'échafaudage de tissu, d'un autre agent antimicrobien, d'un agent de débridement de plaie, d'un agent antimicrobien choisi dans l'ensemble constitué de chlorhexidine, d'un sel de chlorhexidine, d'un triclosane, d'une polymoxine, d'une tétracycline, d'un amino glycoside, d'une rifampicine, d'une bacitracine, d'une érythromycine, d'une néomycine, d'un chloramphénicol, d'une miconazole, d'une quinolone, d'une pénicilline, d'un nonoxynol 9, d'un acide fusidique, d'une céphalosporine, d'une mupirocine, d'une métronidazole, d'une sécropine, d'une protégrine, d'une bactériolcine, d'une défensine, d'une nitrofurazone, d'un mafénide, d'un acyclovir, d'une vancomycine, d'une clindamycine, d'une lincomycine, d'un sulfonamide, d'une norfloxacine, d'une pefloxacine, d'un acide nalidizique, d'un acide oxalique, d'un acide d'énoxacine, d'une ciprofloxacine, de leurs combinaisons et similaires.

11. Procédé selon la revendication 7 dans lequel l'agent antimicrobien est choisi dans l'ensemble constitué de PHMB, d'un dérivé de PHMB qui est un biguanide biodégradable et PEHMB.
